# EUROPÄISCHE PATENTANMELDUNG

(11) **EP 1 843 140 A2**
(43) Veröffentlichungstag der Anmeldung: **10.10.2007**
(21) Anmeldenummer: 07005894.6
(22) Anmeldetag: 22.03.2007
(51) Int. Cl.: G01L 19/00, A61M 1/36

(54) **Anschlusselement zur lösbaren abgedichteten Verbindung eines Fluidleitungssystems mit einem Druckaufnehmer und Druckaufnehmer hierzu**

(30) Priorität: 07.04.2006 DE 102006016846
(71) Anmelder: MESYS GMBH, 30419 Hannover (DE)
(72) Erfinder: Brucksch, Ulrich, 30916 Isernhagen (DE); Hartwig, Rainer, 29313 Hambühren (DE)
(74) Vertreter: Gerstein, Hans Joachim

(57) **Zusammenfassung**

Ein Anschlusselement (1) zur lösbaren abgedichteten Verbindung eines Fluidleitungssystems mit einem Druckaufnehmer (2) zur Detektion positiver und negativer Drücke eines im Anschlusselement (1) befindlichen Fluids mit einem Gehäuse (3), mindestens einem Fluidleitungsanschluss (4a, 4b) an dem Gehäuse (3), einer Messkammer (5) im Gehäuse (3), die in kommunizierender Verbindung zum Fluidleitungsanschluss, (4a, 4b) steht, einer Membran (8) angrenzend an die Messkammer (5), die zur Abdichtung der Messkammer (5) dicht mit dem Gehäuse (3) verbunden ist, und mit einem Anschlussabschnitt (9) an dem Gehäuse (3) zur wiederholt lösbaren Verbindung des Anschlusselementes (1) an einen Druckaufnehmer (2) wird beschrieben. Der Anschlussabschnitt (9) hat einen vom Gehäuse (3) wegweisenden rohrförmigen Anschluss (13) zum Auf- oder Einstecken in ein korrespondierendes Anschlussrohr (17) des Druckaufnehmers (2), wobei der rohrförmige Anschluss (13) in kommunizierender Verbindung mit der Membran (8) steht und mindestens ein Arretierelement (16) zur luftdichten Verbindung des Anschlusselementes (1) mit dem Druckaufnehmer (2) hat.

## Beschreibung

Die Erfindung betrifft ein Anschlusselement zur lösbaren abgedichteten Verbindung eines Fluidleitungssystems mit einem Druckaufnehmer zur Detektion positiver und negativer Drücke eines im Anschlusselement befindlichen Fluids mit einem Gehäuse, mindestens einem Fluidleitungsanschluss an dem Gehäuse, einer Messkammer im Gehäuse, die in kommunizierender Verbindung zum Fluidleitungsanschluss steht, einer Membran angrenzend an die Messkammer, die zur Abdichtung der Messkammer dicht mit dem Gehäuse verbunden ist, und mit einem Anschlussabschnitt an dem Gehäuse zur wiederholt lösbaren Verbindung des Anschlusselementes an einen Druckaufnehmer.

Die Erfindung betrifft weiterhin einen Druckaufnehmer zum Messen des Drucks eines in einer mit einer Membran abgeschlossenen Messkammer eines separaten, lösbar und abgedichtet an den Druckaufnehmer anschließbaren Anschlusselementes, wobei der Druckaufnehmer ein Druckaufnehmergehäuse, einen Drucksensor in dem Druckaufnehmergehäuse und einen Anschlussbereich zum wiederholt lösbaren Befestigen des Anschlusselementes an dem Druckaufnehmergehäuse derart hat, dass das Anschlusselement luftdicht mit dem Druckaufnehmer verbunden ist und zwischen der Membran und dem Drucksensor ein zur Atmosphäre abgedichtetes Luftvolumen ist, so dass ein Druck der Membran über das Luftvolumen auf den Drucksensor wirkt.

Beispielsweise bei der Dialyse oder Hämodialyse besteht ein Bedarf, den Blutdruck in einer Leitung kontinuierlich zu messen und zu überwachen. Aus hygienischen Gründen und zur Entkopplung der Messelektronik von dem Patienten ist hierbei das in einer Messkammer eines separaten Anschlusselementes befindliche fluide Medium durch eine Membran von dem Druckaufnehmer getrennt. Das Anschlusselement ist mit einem Fluidleitungssystem eines Dialyse- oder Hämodialysegerätes gekoppelt.

Derartige Anschlusselemente (Systemelemente) sowie gattungsgemäße Druckaufnehmer sind beispielsweise aus der DE 100 32 616 A1 bekannt. Das Systemelement wird dabei mit Federhaken auf den Druckaufnehmer so aufgesetzt, dass die Membran des Systemelements bündig und luftdicht auf einer Transducermembran eines Drucksensors aufliegt. Die Federhaken greifen dabei in eine am Außenumfang des Gehäuses umlaufende Nut ein.

Weiterhin ist aus der EP 0 685 721 B1 eine Vorrichtung zum Messen des Drucks eines fluiden Mediums bekannt, bei der die Membran des Systemelementes an die Transducermembran des Drucksensors angepresst wird.

Die Messung des positiven oder negativen Fluiddrucks erfordert eine absolut dichte und präzise Befestigung des Anschlusselementes an den Druckaufnehmer. Die Druckmessung während des Dialyse- oder Hämodialysevorgangs ist sicherheitskritisch, so dass ein absolut sicherer Sitz des Anschlusselementes auf dem Druckaufnehmer bei einfachster Bedienung gewährleistet werden muss.

Aufgabe der vorliegenden Erfindung ist es daher, ein verbessertes Anschlusselement zur lösbaren abgedichtet Verbindung eines Fluidleitungssystems mit einem Druckaufnehmer sowie einen verbesserten Druckaufnehmer hierzu zu schaffen, die bei einfachster Bedienung einen festen und sicheren Sitz aufeinander gewährleisten.

Die Aufgabe wird mit dem Anschlusselement der eingangs genannten Art dadurch gelöst, dass der Anschlussabschnitt einen vom Gehäuse wegweisenden rohrförmigen Anschluss zum Auf- oder Einstecken in ein korrespondierendes Anschlussrohr des Druckaufnehmers und mindestens ein Arretierelement zur wiederholt lösbaren Verbindung des Anschlusselementes mit einem Druckaufnehmer hat, wobei der rohrförmige Anschluss in kommunizierender Verbindung mit der Membran steht und zur luftdichten Verbindung des Anschlusselementes mit dem Druckaufnehmer ausgebildet ist.

Im Unterschied zu den herkömmlichen flach aufeinander liegenden Anschlusselementen und Druckaufnehmern wird mit der vorliegenden Erfindung vorgeschlagen, Anschlusselement und Druckaufnehmer mit Hilfe von ineinandergreifenden korrespondierenden rohrförmigen Anschlussabschnitten miteinander zu verbinden. Dabei dienen die Anschlussrohre einerseits zum sicheren und dichten Festlegen von Anschlusselement und Druckaufnehmer aufeinander. Andererseits definieren das innen liegende Anschlussrohr das Luftvolumen zwischen Membran und Drucksensor des Druckaufnehmers. Die Präzision von Anschlusselement und Druckaufnehmer wird damit durch die Fertigung des das Luftvolumen definierenden Anschlussrohrs wesentlich bestimmt und ist nicht von der Bedienung abhängig.

Dadurch, dass die Membran nach Außen im Wesentlichen durch den Anschlussabschnitt abgedeckt und nur über den rohrförmigen Anschluss nach Außen zugängig ist, wird eine Beschädigung der Membran beim Handling des Anschlusselementes vermieden. Zudem wird die Membran so stabilisiert, dass ein Platz in der Membran bei normalen Systemdrücken im Unterschied zu den herkömmlichen freien Membranen sicher verhindert wird.

Durch Handlingfehler ist es bei den herkömmlichen freiliegenden Membranen oftmals zu dem Problem einer Beschädigung bzw. eines Aufplatzens der Membran gekommen, wordurch Blut aus den Anschlussleitungen nach Außen austritt und die Umgebung kontaminiert und ggf. auch den Patienten gefährdet.

Das Arretierelement ist vorzugsweise als Schraubverschluss mit Schraubgewinde ausgeführt. Ein solcher Schraubverschluss kann im Unterschied zu den herkömmlichen störanfälligen Schnappverschlüssen aufgrund der ineinandergreifenden rohrförmigen Anschlussrohre realisiert werden. Mit dem Schraubverschluss kann durch einfachste schnelle Bedienung eine sichere und dichte Verbindung von Anschlusselement und Druckaufnehmer geschaffen werden. Besonders vorteilhaft ist es, wenn als Schraubverschluss ein Luerlockanschluss genutzt wird, wie er in Krankenhäusern üblich ist. Die Bedienung eines solchen Luerlockanschlusses ist aufgrund der weiten Verbreitung in Krankenhäusern gut bekannt. Zudem bietet ein Luerlockanschluss aufgrund der relativ großen Gewindesteigung den Vorteil, dass nur eine geringe Umdrehung zum Verschrauben und Lösen des Anschlussabschnitts erforderlich ist.

Optional ist auch eine auf schnelle und einfachste Weise bedienbare Befestigung des Anschlusselementes an dem Druckaufnehmer mit einem Bajonettverschluss denkbar. Der Bajonettverschluss kann dadurch ausgebildet sein, dass der rohrförmige Anschluss ein sich vom freien Ende in Richtung Membran erstreckenden Längsschlitz und ein rechtwinklig vom Ende des Lenkschlitzes abgehenden Querschlitz hat. Eine knopfartige Erhebung am korrespondierenden Anschlussrohr des Druckaufnehmers wird dann in einen korrespondierenden Längsschlitz geführt und bei Erreichen des Querschlitzes in diesen hineingedreht.

Alternativ kann der rohrförmige Anschluss des Anschlusselementes mindestens eine knopfartige Erhebung zum Einführen in einen Bajonettverschlussschlitz des Druckaufnehmers haben. Denkbar ist auch eine Kombination, wobei der rohrförmige Anschluss des Anschlusselementes einen Längsschlitz und eine knopfartige Erhebung korrespondierend zu einem Längsschlitz und zu einer knopfartigen Erhebung des Anschlussrohrs des Druckaufnehmers hat.

Die Bedienung des Arretierelementes kann dadurch erleichtert werden, dass ein relativ zu dem rohrförmigen Anschluss verdrehbares Überwurfteil mit einem vom Überwurfteil hervorragenden Hebel zur Arretierung des Anschlusselementes an einen Druckaufnehmer vorgesehen ist. Mit Hilfe des Hebels kann eine schnelle und einfache Verriegelung erfolgen. Gleichzeitig zeigt der Riegel durch die Erstreckungsrichtung des Hebels deutlich, ob das Anschlusselement fest mit dem Druckaufnehmer verriegelt ist oder nicht.

Weiterhin ist vorteilhaft, wenn eine Mulde in dem Gehäuse angrenzend an die Membran und diametral gegenüberliegend zu der Messkammer vorgesehen ist. Durch die Mulde kann sich die Membran nach unten in Richtung rohrförmigen Anschluss noch stärker bewegen, als dies durch die Öffnung im rohrförmigen Anschluss selbst möglich ist. Hierdurch wird die Messung positiver Drücke in der Messkammer verbessert.

Der Anschlussabschnitt ist vorzugsweise integral mit dem Gehäuse des Anschlusselementes so geformt, dass dieser unmittelbar angrenzend an die Membran auf das Gehäuse aufgesetzt und luftdicht mit dem Gehäuse so verbunden ist, dass eine Luftdruckübertragung ausschließlich von der Membran über den rohrförmigen Anschluss zu einem anschließbaren Druckaufnehmer erfolgt. Gehäusemembran und Anschlussabschnitt bilden auf diese Weise eine in sich abgeschlossene Dichteeinheit.

Zur Abdichtung der Messkammer und des Luftvolumens zwischen Membran und Druckaufnehmer ist es vorteilhaft, wenn die Membran einseitig und besonders bevorzugt beidseitig jeweils einen umlaufenden Dichtring und das Gehäuse und der Anschlussabschnitt umlaufende Nuten zur Aufnahme korrespondierender Dichtringe der Membran haben.

Auf diese Weise wird eine luft- und fluiddichte Verbindung des Gehäuses, der Membran und des Anschlussabschnittes miteinander erreicht. Die Dichtringe können integral mit der Membran geformt werden, indem bei der Herstellung Materialverdickungen zur Ausbildung von O-Ringen erzeugt werden.

Gehäuse, Membran und Anschlussabschnitt können miteinander verklebt oder verschweißt werden, um eine luft- und fluiddichte Einheit zu schaffen.

Die Aufgabe wird weiterhin mit dem Druckaufnehmer der eingangs genannten Art dadurch gelöst, dass der Anschlussbereich ein Anschlussrohr zur Aufnahme des rohrförmigen Anschlusses des Anschlusselementes ist und Arretierungselemente zur wiederholt lösbaren Verbindung des rohrförmigen Anschlusses des Anschlusselementes an den Anschlussbereich hat.

Korrespondierend zu dem Anschlusselement ist auch bei dem Druckaufnehmer ein rohrförmiger Anschluss vorgesehen, der eine einfache, sichere und dichte Verbindung des Druckaufnehmers mit dem Anschlusselement gewährleistet.

Vorteilhafte Ausführungsformen sind in den Unteransprüchen beschrieben.

Die Erfindung wird anhand eines Ausführungsbeispiels mit den beigefügten Zeichnungen näher erläutert. Es zeigen:
- Figur 1 -: Explosionsansicht eines Anschlusselementes und zugeordneten Druckaufnehmers im Querschnitt;
- Figur 2 -: Querschnittsansicht eines auf einen Druckaufnehmer aufgesetzten Anschlusselementes im Querschnitt.

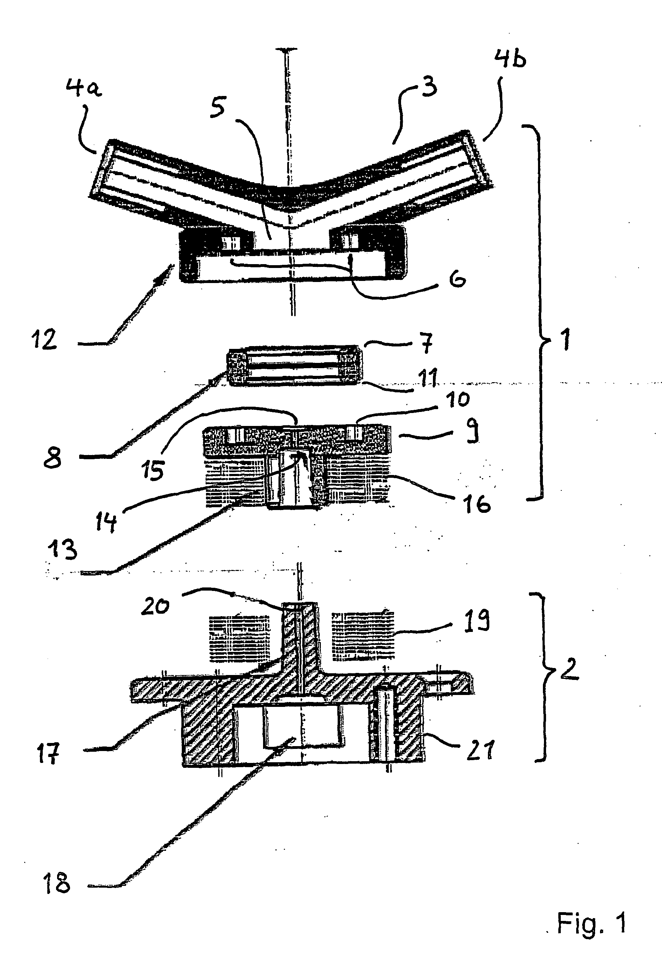

Die Figur 1 lässt eine Explosionsansicht eines Anschlusselementes 1 und eines Druckaufnehmers 2 im Querschnitt erkennen. Das Anschlusselement 1 hat ein Gehäuse 3 mit einem ersten und zweiten Fluidleitungsanschluss 4a, 4b, an die Blutschläuche eines Dialyse- oder Hämodialysegerätes angeschlossen werden können. In dem Zentrum des Gehäuses ist eine Messkammer 5 eingebracht, die in Verbindung mit den Fluidleitungsanschlüssen 4a, 4b steht. Angrenzend an die Messkammer 5 ist eine umlaufende Nut 6 angeordnet, in die ein ebenfalls umlaufender Dichtring 7 einer Membran 8 so eingesteckt werden kann, dass die Membran 8 die Messkammer 5 nach außen hin abdichtet und im Membran 8 sicher und mit verspannter Oberfläche auf der Messkammer 5 sitzt.

Das Anschlusselement 1 hat weiterhin einen Anschlussabschnitt 9, der ebenfalls eine umlaufende Nut 10 zur Aufnahme eines korrespondierenden Dichtrings 11 der Membran 8 aufweist. Damit wird sichergestellt, dass das Luftvolumen zwischen Membran 8 und Druckaufnehmer 2 begrenzt ist und keine Luft seitlich durch die Membran 8 hindurch nach außen weichen kann.

Der Anschlussabschnitt 9 wird mit zwischengelegter Membran 8 auf das Gehäuse 3 aufgesetzt und mit diesem fest, beispielsweise durch Verkleben oder Verschweißen verbunden. Hierzu ist beispielsweise ein Kragen 12 an dem Gehäuse 3 vorgesehen, der den Anschlussabschnitt 9 übergreift und zur luftdichten Verbindung des Anschlussabschnitts 9 und des Gehäuses 3 genutzt werden kann.

Der Anschlussabschnitt 9 hat einen rohrförmigen Anschluss 13, der sich im zusammengebauten Zustand vom Gehäuse 3 und der Anlagefläche des Anschlussabschnitts 9 gesehen nach unten weg erstreckt. Zur Definition des Luftvolumens zwischen Membran 8 und Druckaufnehmer 2 ist eine relativ kleine Bohrung 13 in die Auflagefläche des Anschlussabschnitts 9 eingebracht. Das Luftvolumen sollte möglichst klein sein, um eine hinreichend genaue Messung zu ermöglichen.

Weiterhin ist angedeutet, dass angrenzend an die Membran 8 in der Auflagefläche des Anschlussabschnitts 9 eine Mulde 15 eingebracht ist, in die sich die Membran 8 hineinlegen kann, wenn in der Messkammer 5 ein Überdruck gegenüber der Atmosphäre besteht. Das Luftvolumen zwischen Membran 8 und Druckaufnehmer 2 kann auf diese Weise komprimiert und eine Messung positiver Drücke ermöglicht werden.

Der rohrförmige Anschluss 13 hat ein mit gestrichelten Linien angedeutetes Arretierungselement 16, um das Anschlusselement 1 einfach, sicher und luftdicht auf den Druckaufnehmer 2 aufzusetzen. Hierzu erstreckt sich ein korrespondierendes Anschlussrohr 17 des Druckaufnehmers 2 von einem Drucksensor 18 weg nach oben. Angrenzend an das Anschlussrohr 17 ist an dem Druckaufnehmer 2 ebenfalls ein Arretierelement 19 vorgesehen, dass mit dem Arretierelement 16 des Anschlusselementes 3 zusammenwirkt.

Durch eine relativ kleine Bohrung 20 im Anschlussrohr 17 wird ein möglichst geringes Luftvolumen zwischen Drucksensor 18 und Membran 8 sichergestellt, wenn das Anschlusselement 3 auf den Druckaufnehmer 2 aufgesetzt ist.

Das Arretierungselement 16 kann beispielsweise als Schraubverschluss mit Schraubgewinde oder als Bajonettverschluss in an sich für andere Anwendungen bekannter Weise ausgeführt sein. Dabei ist es vorteilhaft, wenn quer zur Längsachse des rohrförmigen Anschlusses 13 ein Hebel abgeht, mit dessen Hilfe das Arretierungselement um den zum Verriegeln erforderlichen Winkel verdreht werden kann. Vorzugsweise ist das Arretierelement 16 konstruktiv so ausgebildet, dass zum Ver- und Entriegeln eine Drehung um maximal 90° und bevorzugt um etwa 45° erforderlich ist.

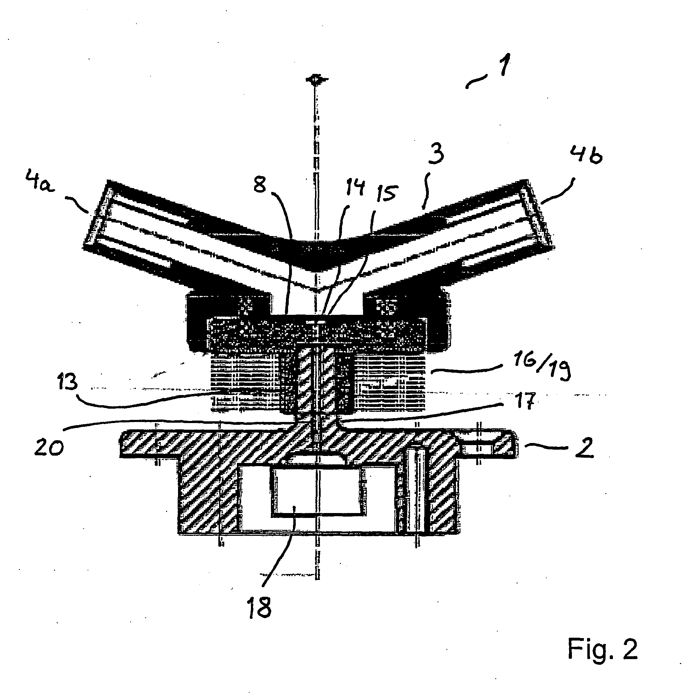

Die Figur 2 lässt eine Querschnittsansicht des Anschlusselementes 1 aus der Figur 1 erkennen, dass auf den Druckaufnehmer 2 aufgesetzt ist. Dabei greifen das Arretierelement 16 des Anschlusselementes 1 in das am Druckaufnehmergehäuse 21 angeformten Arretierungselement 19 des Druckaufnehmers 2 so ein, dass das Anschlusselement 1 fest und dicht auf dem Druckaufnehmer 2 aufsitzt.

Es ist auch erkennbar, dass die Bohrungen 15 und 20 miteinander fluchtend sind und in kommunizierender Verbindung mit dem Drucksensor 18 und der Mulde 15 unterhalb der Membran 8 stehen. Auf diese Weise wird ein kleines definiertes Luftvolumen zwischen Membran 8 und Drucksensor 18 bereitgestellt.

## Patentansprüche

1. Anschlusselement (1) zur lösbaren abgedichteten Verbindung eines Fluidleitungssystems mit einem Druckaufnehmer (2) zur Detektion positiver und negativer Drücke eines im Anschlusselement (1) befindlichen Fluids mit einem Gehäuse (3), mindestens einem Fluidleitungsanschluss (4a, 4b) an dem Gehäuse (3), einer Messkammer (5) im Gehäuse (3), die in kommunizierender Verbindung zum Fluidleitungsanschluss (4a, 4b) steht, einer Membran (8) angrenzend an die Messkammer (5), die zur Abdichtung der Messkammer (5) dicht mit dem Gehäuse (3) verbunden ist, und mit einem Anschlussabschnitt (9) an dem Gehäuse (3) zur wiederholt lösbaren Verbindung des Anschlusselementes (1) an einen Druckaufnehmer (2), **dadurch gekennzeichnet, dass** der Anschlussabschnitt (9) einen vom Gehäuse (3) wegweisenden rohrförmigen Anschluss (13) zum Auf- oder Einstecken in ein korrespondierendes Anschlussrohr (17) des Druckaufnehmers (2) und mindestens eine Arretierelement (16) zur wiederholt lösbaren Verbindung des Anschlusselementes (1) mit dem Druckaufnehmer (2) hat, wobei der rohrförmige Anschluss (13) in kommunizierender Verbindung mit der Membran (8) steht und zur luftdichten Verbindung des Anschlusselementes (1) mit dem Druckaufnehmer (2) ausgebildet ist.

2. Anschlusselement (1) nach Anspruch 1, **dadurch gekennzeichnet, dass** das Arretierelement (16) als Schraubverschluss mit Schraubgewinde ausgeführt ist.

3. Anschlusselement (1) nach Anspruch 2, **dadurch gekennzeichnet, dass** das Arretierelement (16) als Luerlock-Anschluss ausgebildet ist.

4. Anschlusselement (1) nach Anspruch 1, **dadurch gekennzeichnet, dass** das Arretierelement (16) als Bajonettverschlusselement ausgebildet ist.

5. Anschlusselement (1) nach Anspruch 4, **dadurch gekennzeichnet, dass** der rohrförmige Anschluss (13) mindestens einen sich vom freien Ende in Richtung Membran (8) erstreckenden Längsschlitz und einen rechtwinklig vom Ende des Längsschlitzes abgehenden Querschlitz hat.

6. Anschlusselement (1) nach Anspruch 4, **dadurch gekennzeichnet, dass** der rohrförmige Anschluss (13) mindestens eine knopfartige Erhebung zum Einführen in einen Bajonettverschlussschlitz des Druckaufnehmers (2) hat.

7. Anschlusselement (1) nach einem der vorhergehenden Ansprüche, **dadurch gekennzeichnet, dass** das Arretierelement (16) ein relativ zu dem rohrförmigen Anschluss (13) verdrehbares Überwurfteil mit einem vom Überwurfteil hervorragenden Hebel zur Arretierung des Anschlusselementes (1) an einen Druckaufnehmer (2) hat.

8. Anschlusselement (1) nach einem der vorhergehenden Ansprüche, **gekennzeichnet durch** eine Mulde (15) in dem Gehäuse (3) angrenzend an die Membran (8) und diametral gegenüberliegend zu der Messkammer (5).

9. Anschlusselement (1) nach einem der vorhergehenden Ansprüche, **dadurch gekennzeichnet, dass** der Anschlussabschnitt (9) unmittelbar angrenzend an die Membran (8) auf das Gehäuse (3) aufgesetzt und luftdicht mit dem Gehäuse (3) so verbunden ist, dass eine Luftdruckübertragung ausschließlich von der Membran (8) über den rohrförmigen Anschluss (13) zu einem anschließbaren Druckaufnehmer (2) erfolgt.

10. Anschlusselement (1) nach einem der vorhergehenden Ansprüche, **dadurch gekennzeichnet, dass** die Membran (8) einseitig einen umlaufenden Dichtring (7) und das Gehäuse (3) umlaufenden Nuten (6) zur Aufnahme des Dichtrings (7) der Membran (8) und zur luft- und fluiddichten Verbindung des Gehäuses (3) mit der Membran (8) hat.

11. Anschlusselement (1) nach einem der vorhergehenden Ansprüche, **dadurch gekennzeichnet, dass** die Membran (8) beidseitig jeweils einen umlaufenden Dichtring (7, 11) und das Gehäuse (3) und der Arnachlussabschnitt (9) umlaufende Nuten (6, 10) zur Aufnahme korrespondierender Dichtringe (7, 11) der Membran (8) und zur luft- und fluiddichten Verbindung des Gehäuses (3), der Membran (8) und des Anschlussabschnitts (9) miteinander haben.

12. Anschlusselement (1) nach einem der vorhergehenden Ansprüche, **dadurch gekennzeichnet, dass** das Gehäuse (3), die Membran (8) und der Anschlussabschnitt (9) miteinander verklebt oder verschweißt sind.

13. Druckaufnehmer (2) zum Messen des Drucks eines in einer mit einer Membran (8) abgeschlossenen Messkammer eines separaten, lösbar und abgedichtet an den Druckaufnehmer (2) anschließbaren Anschlusselementes (1) nach einem der vorhergehenden Ansprüche, wobei der Druckaufnehmer (2) ein Druckaufnehmergehäuse (21), einen Drucksensor (18) in dem Druckaufnehmergehäuse und einen Anschlussbereich zum wiederholt lösbaren Befestigen des Anschlusselementes (1) an dem Druckaufnehmergehäuse derart hat, dass das Anschlusselement (1) luftdicht mit dem Druckaufnehmer (2) verbunden ist und zwischen der Membran (8) und dem Drucksensor (18) ein zur Atmosphäre abgedichtetes Luftvolumen ist, so dass ein Druck der Membran (8) über das Luftvolumen auf den Drucksensor (18) wirkt, **dadurch gekennzeichnet, dass** der Anschlussbereich ein Anschlussrohr (17) zur Aufnahme des rohrförmigen Anschlusses (13) des Anschlusselementes (1) ist und mindestens ein Arretierelement (16) zur wiederholt lösbaren Verbindung des rohrförmigen Anschlusses (13) des Anschlusselementes (1) an den Anschlussbereich hat.

14. Druckaufnehmer (2) nach Anspruch 12, **dadurch gekennzeichnet, dass** das Arretierelement (16) am Außenumfang des Anschlussrohres angeordnet ist, um ein auf das Anschlussrohr (17) aufgesteckten rohrförmigen Anschluss (13) des Anschlusselementes (1) aufzunehmen, und der Innenraum des Anschlussrohrs (17) das Luftvolumen definiert.

15. Druckaufnehmer (2) nach Anspruch 12, **dadurch gekennzeichnet, dass** das Anschlussrohr (17) zur Aufnahme eines rohrförmigen Anschlusses (13) eines Anschlusselementes (1) im Innenraum des Anschlussrohres (17) vorgesehen ist, wobei der Innenraum des rohrförmigen Anschlusses (13) das Luftvolumen definiert.

16. Druckaufnehmer (2) nach einem der Ansprüche 12 bis 14, **dadurch gekennzeichnet, dass** das Anschlussrohr (17) einen Schraubverschluss oder einen Bajonettverschluss hat.
